# EUROPEAN PATENT APPLICATION

(11) **EP 4 613 760 A1**
(43) Date of publication of application: **10.09.2025**
(21) Application number: 23886075.3
(22) Date of filing: 17.10.2023
(51) Int. Cl.: C07K 14/495, C07K 1/02, C07K 1/20, C07K 1/18, C07K 1/34

(54) **METHOD FOR PURIFYING TGF-BETA3 PROTEIN**

(30) Priority: 04.11.2022 KR 20220146295
(71) Applicant: Daewoong Pharmaceutical Co., Ltd., Hwaseong-si, Gyenoggi-do 18623 (KR)
(72) Inventor: LEE, Sungjin, Seoul 06170 (KR); LEE, Sangeun, Seoul 06170 (KR); KIM, Boo Young, Seoul 06170 (KR); LEE, Ho-Bin, Seoul 06170 (KR); KIM, Nam Hyun, Seoul 06170 (KR); KANG, Seung-hoon, Seoul 06170 (KR)
(74) Representative: Plasseraud IP
(86) International application number: PCT/KR2023/016023
(87) International publication number: WO 2024/096378

(57) **Abstract**

The present invention relates to a pretreatment method for isolating a TGF-β3 protein from *E. coli* and purifying same with high efficiency and high purity, and a TGF-β3 protein purification method comprising the pretreatment method. The pretreatment method and the purification method comprising same, according to the present invention, use *E. coli,* and thus are expected to have simpler processes and higher productivity than animal cell culture methods such that highly-pure TGF-β3 protein can be isolated from *E*. *coli* and purified with high efficiency and high purity.

## Description

### [Technical Field]

This application claims the priority right of the Korea Patent Application No. 10-2022-0146295, filed on November 4, 2022, the entire disclosure of which is incorporated herein by reference.

The present invention relates to a pretreatment method for isolating and purifying TGF-β3 protein from *E. coli* with high efficiency and high purity, and a TGF-β3 protein purification method including the same.

### [Background Art]

TGF-β3, a subtype of transforming growth factor-β (TGF-β), is essential for various biological processes, including endoderm development, organogenesis, epithelial proliferation, extracellular matrix synthesis, and immune responses. Essentially, TGF-β3 engages in the TGF-β1/Smad signaling pathway, thereby stimulating mesenchymal lineage cells, inhibiting epithelial or neuroectodermal lineage cells, and regulating skin wound repairing and remodeling and potential scarring.

Bioactive human TGF-β3 is difficult to produce on a large scale in both prokaryotic and eukaryotic expression systems. The latter has low yield and is methodologically complex and expensive, while the former produces inactive inclusion bodies. Therefore, a novel, scalable, time-saving, and cost-saving production platform is urgently needed to produce high-purity TGF-β3.

### [Related Art Documents]

### [Patent Documents]

(Patent Document 1) KR 10-0991203 B1 (published on October 26, 2010)
(Patent Document 2) KR 10-1443257 B1 (published on September 16, 2014)

### [Disclosure]

### [Technical Problem]

The present inventors have made efforts to provide a novel, scalable, time-saving, and cost-saving production platform for producing high-purity TGF-β3, and as a result, have confirmed that high-purity TGF-β3 can be isolated and purified with high efficiency when using the pretreatment method of the present invention and the purification method including the same, thereby completing the present invention.

Therefore, an object of the present invention is to provide a pretreatment method for isolating and purifying TGF-β3 protein from *E. coli* with high efficiency and high purity, and a TGF-β3 protein purification method including the same.

### [Technical Solution]

The present invention provides a pretreatment method for purifying TGF-β3 protein, including: i) a step of recovering *E. coli* from an *E. coli* culture medium;
ii) a step of disrupting the recovered *E. coli* to obtain a TGF-β3 inclusion body;
iii) a step of washing the inclusion body;
iv) a step of solubilizing the washed inclusion body; and
v) a step of refolding the solubilized inclusion body.

According to one preferred embodiment of the present invention, the disrupting in Step ii) is performed one to five times at a pressure of 800 to 1200 bar.

According to one preferred embodiment of the present invention, the washing in Step iii) is performed one to five times.

According to one preferred embodiment of the present invention, the washing in Step iii) includes a step of washing with a surfactant and then washing with distilled water.

According to one preferred embodiment of the present invention, the solubilizing in Step iv) is performed with a buffer containing one or more selected from the group consisting of urea, 1,4-dithiothreitol (DTT), and tris(hydroxymethyl)aminomethane.

According to one preferred embodiment of the present invention, the solubilizing in Step iv) is performed with a buffer having a pH of 7 to 9.

According to one preferred embodiment of the present invention, the solubilizing in Step iv) is performed with a buffer for 1 to 24 hours.

According to one preferred embodiment of the present invention, the refolding in Step v) is performed with a buffer containing one or more selected from the group consisting of N-cyclohexyl-2-aminoethanesulfonic acid (CHES), NaCl, reduced glutathione, oxidized glutathione, 3-[(3-cholamidopropyl)dimethylammonio]propane-1-sulfonate (CHAPS), L-arginine, and D-sorbitol.

According to one preferred embodiment of the present invention, the refolding in Step v) is performed with a buffer having a pH of 8.5 to 10.5.

According to one preferred embodiment of the present invention, the refolding in Step v) is performed with a buffer for 1 to 12 days.

According to one preferred embodiment of the present invention, the refolding in Step v) is performed so that the final concentration becomes 0.1 to 2.0 g/L.

In addition, the present invention provides a TGF-β3 protein purification method including: i) a step of recovering *E. coli* from an *E. coli* culture medium;
ii) a step of disrupting the recovered *E. coli* to obtain a TGF-β3 inclusion body;
iii) a step of washing the inclusion body;
iv) a step of solubilizing the washed inclusion body;
v) a step of refolding the solubilized inclusion body;
vi) a step of purifying a solution containing the refolded TGF-β3; and
vii) a step of filtering the purified solution.

According to one preferred embodiment of the present invention, the purifying in Step vi) includes:
a) a step of performing hydrophobic interaction chromatography as primary purification;
b) a step of performing multimodal chromatography as secondary purification; and
c) a step of performing cation exchange chromatography as a tertiary purification.

According to one preferred embodiment of the present invention, the filtering in Step vii) is ultrafiltration/diafiltration.

Hereinafter, the present invention will be described in more detail.

The present inventors have derived an optimal pretreatment step for *E*. *coli* to isolate and purify high-purity TGF-β3, and the TGF-β3 protein purification method including the pretreatment step of the present invention can isolate and purify high-purity TGF-β3 from *E. coli* with high efficiency.

**In** particular, the present inventors have derived a method for isolating and purifying high-purity TGF-β3, and further completed a process method that can reduce the process time, thereby providing a more efficient TGF-β3 protein purification method. Specifically, when the pretreatment method and purification method of the present invention are used, 1 to 1.5 g of TGF-β3 protein can be obtained by performing a 2.5 L *E. coli* culture.

Therefore, the present invention may provide a pretreatment method for purifying TGF-β3 protein, including: i) a step of recovering *E. coli* from an *E. coli* culture medium;
ii) a step of disrupting the recovered *E. coli* to obtain a TGF-β3 inclusion body;
iii) a step of washing the inclusion body;
iv) a step of solubilizing the washed inclusion body; and
v) a step of refolding the solubilized inclusion body.

According to one preferred embodiment of the present invention, the disrupting in Step ii) may be performed one to five times at a pressure of 800 to 1200 bar. More preferably, the disrupting in Step ii) may be performed two to three times at a pressure of 1000 bar.

According to one preferred embodiment of the present invention, the washing in Step iii) may be performed one to five times. More preferably, the disrupting in Step ii) may be performed two to three times.

According to one preferred embodiment of the present invention, the washing in Step iii) may include a step of washing with a surfactant and then washing with distilled water.

The surfactant may be Tween 20.

The washing with a surfactant may be performed for 1 to 24 hours, more preferably, for 2 to 18 hours.

The washing with distilled water may be performed one to three times, more preferably, one to two times.

The washing with distilled water may be performed for 1 to 24 hours, more preferably, for 2 to 18 hours.

According to one preferred embodiment of the present invention, the solubilizing in Step iv) may be performed with a buffer containing one or more selected from the group consisting of urea, 1,4-dithiothreitol (DTT), and tris(hydroxymethyl)aminomethane.

According to one preferred embodiment of the present invention, the solubilizing in Step iv) may be performed with a buffer having a pH of 7 to 9. More preferably, the buffer may have a pH of 7.5 to 8.5.

According to one preferred embodiment of the present invention, the solubilizing in Step iv) may be performed with a buffer for 1 to 24 hours. More preferably, the solubilizing in Step iv) may be performed for 3 to 24 hours.

According to one preferred embodiment of the present invention, the refolding in Step v) may be performed with a buffer containing one or more selected from the group consisting of N-cyclohexyl-2-aminoethanesulfonic acid (CHES), NaCl, reduced glutathione, oxidized glutathione, 3-[(3-cholamidopropyl)dimethylammonio]propane-1-sulfonate (CHAPS), L-arginine, and D-sorbitol. More preferably, the refolding in Step v) may be performed with a buffer containing one or more selected from the group consisting of CHES, NaCl, reduced glutathione, and oxidized glutathione.

According to one preferred embodiment of the present invention, the refolding in Step v) may be performed with a buffer having a pH of 8.5 to 10.5. More preferably, the buffer may have a pH of 9 to 10.

According to one preferred embodiment of the present invention, the refolding in Step v) may be performed with a buffer for 1 to 12 days. More preferably, the refolding in Step v) may be performed with a buffer for 3 to 12 days.

According to one preferred embodiment of the present invention, the refolding in Step v) may be performed so that the final concentration becomes 0.1 to 2.0 g/L. Preferably, the refolding in Step v) may be performed so that the final concentration becomes 0.1 to 1.5 g/L, more preferably, the refolding in Step v) may be performed so that the final concentration becomes 0.1 to 0.5 g/L, and most preferably, the refolding in Step v) may be performed so that the final concentration becomes 0.1 to 0.3 g/L.

In addition, the present invention provides a TGF-β3 protein purification method including: i) a step of recovering *E. coli* from an *E. coli* culture medium;
ii) a step of disrupting the recovered *E. coli* to obtain a TGF-β3 inclusion body;
iii) a step of washing the inclusion body;
iv) a step of solubilizing the washed inclusion body;
v) a step of refolding the solubilized inclusion body;
vi) a step of purifying a solution containing the refolded TGF-β3; and
vii) a step of filtering the purified solution.

Since Steps i) to v) are identical to the steps included in the above-described pretreatment method, the description thereof is replaced by the above description.

According to one preferred embodiment of the present invention, the purifying in Step vi) includes:
a) a step of performing hydrophobic interaction chromatography as primary purification;
b) a step of performing multimodal chromatography as secondary purification; and
c) a step of performing cation exchange chromatography as a tertiary purification.

According to one preferred embodiment of the present invention, the filtering in Step vii) is ultrafiltration/diafiltration.

### [Advantageous Effects]

When the pretreatment method of the present invention and the purification method including the same are used, since *E. coli* is used, the process is simple and high productivity can be expected compared to an animal cell culture method and therefore high-purity TGF-β3 protein can be isolated and purified with high efficiency from *E. coli.*

### [Description of Drawings]

FIG. 1 shows the results of a sodium dodecyl sulfate-polyacrylamide gel electrophoresis (SDS-PAGE) analysis according to solubilization time. A band corresponding to the size of TGF-monomer was observed, and no change in pattern was observed according to solubilization time.
FIG. 2 shows the results of an SDS-PAGE analysis according to refolding concentration and time. A band corresponding to the size of TGF-dimer was observed, and no change in pattern was observed according to refolding concentration or time.
FIG. 3 shows the results of an SDS-PAGE analysis for the TGF-β3 standard solution (lane 1) and the target protein TGF-β3 (lane 2). It was confirmed that the target protein of the present invention was purified with high purity (M: protein marker).

### [Modes of the Invention]

Hereinafter, the present invention will be described in more detail through examples. These examples are only intended to illustrate the present invention, and it is obvious to those skilled in the art that the scope of the present invention is not limited by these examples.

### [Example 1]

### Obtaining E. coli cells

An *E. coli* BL21 (DE3) strain transformed with a pTT-TGFβ3 plasmid was subjected to seed culture and main culture, and the main culture process solution was centrifuged to recover *E. coli* cells. The recovered cells were added to Buffer A (20 mM Tris, 5 mM ethylenediaminetetraacetic acid (EDTA), pH 8.0±0.2) and then suspended.

### [Example 2]

### Cell disruption

### <2-1> Setting the number of cell disruptions

Cells in the cell recovery solution of [Example 1] were disrupted a total of three times at a pressure of 1000 bar using a high-pressure disruptor. During the disruption process, 1 mL of each of the culture solution obtained before disruption, the culture solution after the first disruption, the culture solution after the second disruption, and the culture solution after the third disruption was sampled, and the optical density (OD) at 600 nm was measured. In addition, when obtaining the culture solution after disruption, the weight of the culture solution obtained at each step was measured. The disruption rate was calculated using the weight of the culture solution and the OD measured at 600 nm as in [Mathematical Formula 1] below. Cell disruption rate (%) = 100 - {(Cell concentration in cell lysate (OD600)) / (Cell concentration before cell disruption (in cell recovery solution) (OD600))} × 100

As a result, as shown in [Table 1] below, it was confirmed that the disruption rate increased approximately 13% during the second disruption and approximately 4% during the third disruption.

**[Table 1]**

| Disruption step | Dilution factor | OD₆₀₀ | Total volume (kg) | Dilution factor x OD X W | Disruption rate (%) |
|---|---|---|---|---|---|
| Before disruption | 100 | 0.5944 | 5.48 | 325.7 | 0 |
| First | 50 | 0.5086 | 5.68 | 144.4 | 55.7 |
| Second | 50 | 0.3344 | 6.19 | 103.5 | 68.2 |
| Third | 50 | 0.269 | 6.74 | 90.7 | 72.2 |

### <2-2> Cell disruption process

The cell recovery solution of [Example 1] was injected into a cell disruptor, and the first cell disruption was performed at a pressure of 1000±200 bar. The first cell lysate was collected in a 5 L beaker, and the collected cell lysate was stirred and stored. The first cell lysate was used to measure the cell concentration.

The cell disruption rate was calculated according to [Mathematical Formula 1], and the numerical value was confirmed.

The first cell lysate was injected into the cell disruptor, and the second cell disruption was performed at a pressure of 1000±200 bar. The second cell lysate was collected in a 10 L beaker, and the collected cell lysate was stirred and stored. The second cell lysate was used to measure the cell concentration. The cell disruption rate was calculated according to [Mathematical Formula 1], and the numerical value was confirmed.

### [Example 3]

### Washing of inclusion body

### <3-1> Setting of washing conditions

As a washing step to remove host cell protein (HCP) and host cell DNA (HCD), a comparative experiment was conducted according to washing buffer, washing step, and washing time.

The washing buffers were as follows: i) 20 mM Tris, 5 mM EDTA, pH 8.0; ii) 20 mM Tris, 5 mM EDTA, 1% Tween 20 (v/v) pH 8.0; iii) 20 mM Tris, 5 mM EDTA, 1.5% Tween 20 (v/v) pH 8.0; and iv) 20 mM Tris, 5 mM EDTA, 1% Triton X-100 (v/v) pH 8.0.

The cell lysates of [Example 2] were centrifuged at 8000 rpm for 30 minutes to obtain inclusion bodies (IB). The weight of the obtained IB was measured, and the IB was divided into 12 equal parts, and suspended in 400 mL of washing buffer prepared according to the respective conditions of [Table 2] below. After washing for the appropriate time (16 hours or 2 hours) for each condition, the first-washed IB was obtained by centrifugation at 8000 rpm for 30 minutes. The supernatant obtained at this time was stored at -20 °C or below for analytical testing. Thereafter, the first-washed IB was suspended in 400 mL of distilled water (DW), and the second washing was performed for the appropriate time according to the conditions of [Table 2]. After the second washing time was over, the washed IB was obtained by centrifugation at 8000 rpm for 30 minutes. The supernatant obtained at this time was also stored at -20 °C or below for analytical testing.

**[Table 2]**

| # | Tween 20 content in first washing detergent (%, v/v) | Time of first washing (Washing buffer) | Time of second washing (DW) |
|---|---|---|---|
| 1 | 1 | 2 hr | 2 hr |
| 2 | 1 | 2 hr | 16 hr |
| 3 | 1.5 | 2 hr | 2 hr |
| 4 | 1.5 | 2 hr | 16 hr |
| 5 | 1 | 16 hr | 2 hr |
| 6 | 1 | 16 hr | 16 hr |
| 7 | 1.5 | 16 hr | 2 hr |
| 8 | 1.5 | 16 hr | 16 hr |
| 9 | 0 | 16 hr | 16 hr |
| 10 | 0 | 16 hr | 2 hr |
| 11 | 1 (Triton X-100, control) | 16 hr | 16 hr |

For the supernatant obtained after the first washing, the residual HCP and HCD were analyzed to evaluate the impurity removal ability in the first washing process. In addition, since the second washing process using DW is a step for removing the detergent used in the first washing, an analytical test for residual Tween 20 was performed with the analytical samples except for the samples for Conditions 9, 10, and 11 that did not contain Tween 20

As a result, as shown in [Table 3] below, it was confirmed that after the first washing in which a detergent containing Tween 20 was used, more than 99% of HCD was removed under all conditions regardless of detergent concentration or washing time, and a greater amount of HCD was removed compared to the condition of using Triton X-100 as the detergent. It was also confirmed that a greater amount of HCP was removed when Tween 20 was used as the detergent compared to the condition of using Triton X-100 regardless of concentration and time. It was confirmed that when the washing buffers did not contain a detergent (Conditions 9 and 10), the removal ability for HCP was better than that of the existing conditions, but the removal ability for HCD was similar to that of the control, and the removal ability was reduced compared to the washing conditions of containing Tween 20.

**[Table 3]**

| Sample | HCD | | HCP | |
|---|---|---|---|---|
| | HCD (ng/mL) | Residual rate (%) | HCP (ng/mL) | Residual rate (%) |
| Pre-wash | 259,613.79 | N/A | 143,166.16 | N/A |
| 1^{st} wash #1 | 2,275.78 | 0.88 | 5,210.32 | 3.64 |
| 1^{st} wash #2 | 2,464.27 | 0.95 | 5,723.43 | 4 |
| 1^{st} wash #3 | 1,878.89 | 0.72 | 6,206.17 | 4.33 |
| 1^{st} wash #4 | 1,799.07 | 0.69 | 5,100.05 | 3.56 |
| 1^{st} wash #5 | 1,388.82 | 0.53 | 5,890.34 | 4.11 |
| 1^{st} wash #6 | 1,791.31 | 0.69 | 5,977.06 | 4.17 |
| 1^{st} wash #7 | 2,228.83 | 0.86 | 10,220.41 | 7.14 |
| 1^{st} wash #8 | 2,076.41 | 0.8 | 11,292.72 | 7.89 |
| 1^{st} wash #9 | 3,604.74 | 1.39 | 11,998.43 | 8.38 |
| 1^{st} wash #10 | 3,015.73 | 1.16 | 4,060.20 | 2.84 |
| 1^{st} wash #11 | 3,251.60 | 1.25 | 15,550.69 | 10.86 |

In addition, it was confirmed that more than 99.8% of Tween 20 was removed under all conditions regardless of washing time and the number of times washing when DW was used.

Therefore, the final washing process was set to perform washing with 1% to 1.5% Tween 20 for 2 to 16 hours in the first washing and 2 to 16 hours in the second washing.

### <3-2> Washing process

For first inclusion body washing, the cell lysates of [Example 2] were centrifuged at 4 °C and 8,000 rpm for 30 minutes. The inclusion bodies recovered through centrifugation were added to Buffer B (20 mM Tris, 5 mM EDTA, 1% (v/v) Tween 20, pH 8.0±0.2) and washed by stirring at room temperature for one day.

For second inclusion body washing, the first inclusion body washing process solution was centrifuged at 4 °C and 8,000 rpm for 30 minutes. The inclusion bodies recovered through centrifugation were added to DW to perform second inclusion body washing by stirring at 300 rpm for 2±1 hours at room temperature.

The secondary inclusion body washing process solution was centrifuged at 4 °C and 8000 rpm for 30 minutes, the inclusion bodies were finally recovered, and the weight was measured.

### [Example 4]

### Solubilization

### <4-1> Setting the solubilization time

The washed inclusion bodies (IB) obtained in [Example 3] were added to a buffer (6 M urea, 100 mM DTT, 50 mM Tris, pH 8.0), and solubilization was performed for 24 hours. After 3 hours, 6 hours, 9 hours, and 24 hours after the start of solubilization, 1 mL of the solubilized solution was sampled and used for analysis tests. Sample analysis was performed by concentration analysis (twice) using a 660 nm assay kit and SDS-PAGE to confirm whether there was a statistical trend over time for the measured sample concentration.

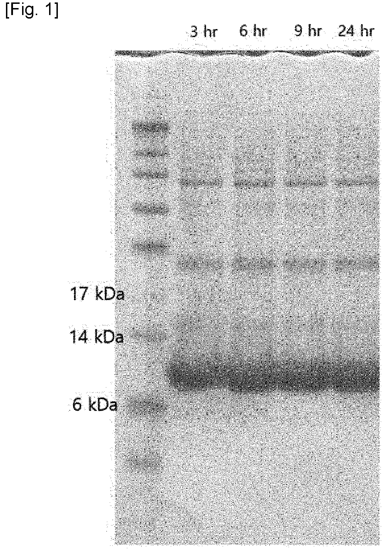

As a result, the p-value of the regression analysis result for the concentration measured twice was 0.2674 and 0.6116, confirming that the concentration of the solubilized solution had no statistical significance over time. Based on these results, it was determined that there was no effect on the sample concentration when the solubilization process is performed for a time period between 3 to 24 hours. In addition, the results of SDS-PAGE analysis confirmed that the band corresponded to the size of a TGF-β3 monomer due to solubilization, and no change in the SDS-PAGE pattern was observed over time, confirming that solubilization time had no effect (FIG. 1).

### <4-2> Solubilization process

The entire amount of the inclusion bodies obtained from the 2.5 L culture medium was added to 1.2 L of Buffer C (6 M Urea, 100 mM DTT, 50 mM Tris, pH 8.0±0.2). The solubilizing solution was stirred at 200 rpm for 16±4 hours to solubilize.

After the reaction was completed, 5 mL of the solubilized solution was sampled twice in 15 mL conical tubes, and one of them was frozen (-20±5 °C). The sampled 5 mL of the solubilized solution was used for protein concentration analysis (660 nm protein assay).

### [Example 5]

### Refolding

### <5-1> Setting of refolding conditions

A total of 4 L of refolding buffer containing 0.7 M CHES, 1 M NaCl, 2 mM GSH, 0.4 mM GSSG, and pH 9.5 was prepared. When preparing the buffer, the pH was first adjusted to 9.2, and then left in a low temperature room for O/N, and then the pH was adjusted to 9.5. The prepared refolding buffer was divided into six 500 mL aliquots. Based on the concentration of the solution subjected to solubilization for 24 hours, the solubilizing solution was added so that the final refolding concentration was 0.1, 0.3, 0.5, 1.0, 1.5, and 2.0 g/L. Before adding the solubilizing solution, the volume of the solubilizing solution to be added was first removed from the divided refolding solution, and then the solubilizing solution was added so that the final volume was maintained at 500 mL. After 0, 1, 3, 5, 7, and 12 days from the start of refolding, 1 mL of the refolding solution was sampled, and 37% HCl in a volume approximately 5% of the sample volume was added to the sampled refolding solution to terminate the refolding reaction. After the refolding reaction was terminated, centrifugation was performed at 13,000 rpm for 30 minutes, and the supernatant was stored at -20 °C or lower and used for analysis. The refolding rate was analyzed by calculating the main peak area of reverse phase high-performance liquid chromatography (RP-HPLC) for the samples that had undergone the reaction for each refolding period. In addition, changes in the band patterns of the monomers and dimers over time were confirmed through SDS-PAGE.

The RP-HPLC analysis was performed on the refolding process solution sampled for each concentration and time. The percent area (%Area) of the dimer peak on the RP-HPLC for each condition is as shown in [Table 4] below.

**[Table 4]**

| | 0.1 g/L | 0.3 g/L | 0.5 g/L | 1.0 g/L | 1.5 g/L | 2.0 g/L |
|---|---|---|---|---|---|---|
| Day 0 | 7.58 | 7.80 | 15.14 | 12.32 | 7.66 | 4.76 |
| Day 3 | 53.62 | 60.56 | 59.17 | 42.36 | 28.27 | N/A |
| Day 5 | 55.24 | 60.49 | 57.35 | 40.03 | 28.61 | |
| Day 7 | 55.81 | 61.03 | 60.11 | 43.17 | 34.04 | |
| Day 12 | 55.90 | 63.01 | 62.51 | 42.90 | 30.06 | |

As a result, it was observed that at concentrations of 1.0 g/L or higher, a large amount of aggregates were formed during the refolding reaction, and thus the process solution became very turbid. It was confirmed that when refolding was performed at a concentration of 0.5 g/L or lower, a dimer efficiency of about 55% to 60% was observed, and when additional refolding was performed from day 3 to day 12, the dimer efficiency was increased by up to about 3%p.

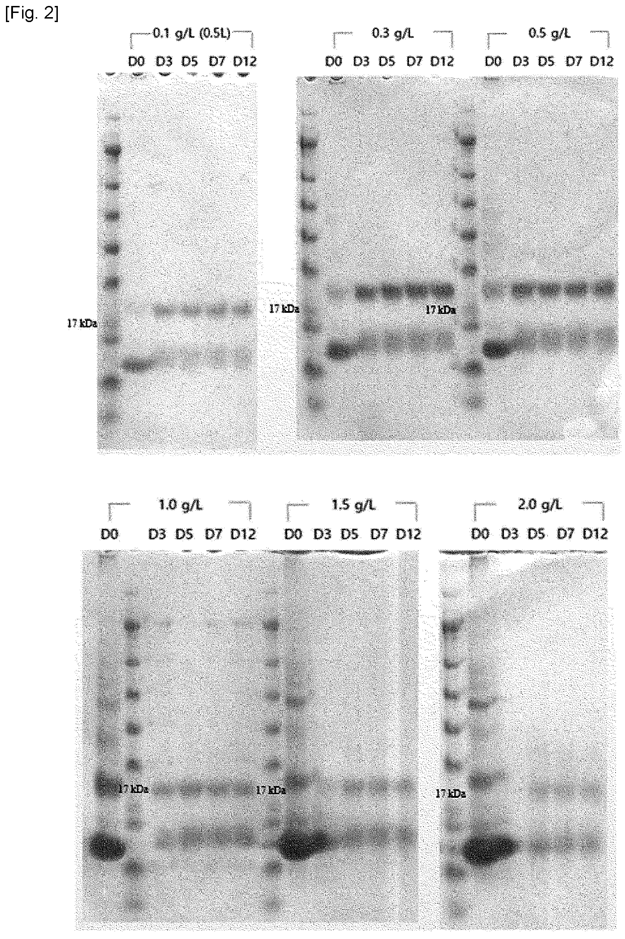

In addition, the dimer efficiency for each sample was confirmed using SDS-PAGE. No change in the SDS-PAGE pattern was observed, confirming that refolding concentration and time had no effect (FIG. 2).

### <5-2> Refolding process

The amount of solubilizing solution input for the refolding process with a protein concentration of 0.1 g/L was calculated according to [Mathematical Formula 2] below. Solubilizing solution input amount (L) = (Refolding process solution volume (L) × 0.1 g/L)/(Protein concentration of solubilizing solution (g/L))

After removing the volume of Buffer D equivalent to the input volume of the solubilizing solution derived from [Mathematical Formula 2] from a 50 L mobile tank, the same volume of the solubilizing solution was input. The refolding process was completed after approximately 72 hours of refolding. The solution that had undergone refolding for three days was pretreated by adjusting the pH to 2.0±0.2 with a hydrochloric acid solution, and then filtered using a capsule filter.

The final filtrate was divided into two 5 mL aliquots in 15 mL conical tubes and used for the following process solution analysis (total protein concentration, 660 nm protein assay). The remaining sample after the process solution analysis was frozen and stored (-20±5 °C). The final filtrate was stored at a low temperature until used as the Purification 1 injection sample.

### [Example 6]

### Purification 1_Hydrophobic chromatography

Buffer E (4 M Urea, 20 mM Na-AcOH, 0.1 M NaCl, pH 4.0±0.2) was injected into the column in an amount of 2 column volumes (CV) or more to perform equilibration. The entire volume of the pretreated refolding process solution was injected into the column. An unbound wash was performed by injecting 10 CV of Buffer E into the column.

Washing was performed by injecting 4 CV of Buffer F (20 mM Tris, pH 8.0±0.2) into the column.

Elution was performed by injecting 3 CV of Buffer G (4 M Urea, 20 mM Tris, pH 9.5±0.2) into the column. The entire amount of 3 CV was recovered as an eluate from the time of injecting Buffer G.

At the end of elution, 5 mL of the elution fraction was divided into 15 mL conical tubes and used for the following process solution analyses: purity (SDS-PAGE), purity (RP-HPLC) (ion pair chromatography (IPC)), and concentration (660 nm protein assay).

### [Example 7]

### Purification 2_Multimodal chromatography

Buffer G (4 M Urea, 20 mM Tris-HCl, pH 9.5±0.2) was injected into the column in an amount of 2 CV or more to perform equilibration. The entire amount of the Purification 1 eluate was injected into the column. An unbound wash was performed by injecting 5 CV of Buffer G into the column.

Washing was performed by injecting 10 CV of Buffer H (4 M Urea, 20 mM Tris-HCl, 0.2 M NaCl, pH 9.5±0.2) into the column.

Elution was performed by injecting 3 CV of Buffer I (4 M Urea, 20 mM Na-AcOH, pH 4.0±0.2) into the column. Eluate recovery was started from the time of injecting 0.5 CV of Buffer I into the column and continued until 3 CV flowed.

At the end of elution, 5 mL of the elution fraction was divided into 15 mL conical tubes and used for the following process solution analyses: purity (SDS-PAGE), purity (RP-HPLC) (IPC), and concentration (660 nm protein assay).

### [Example 8]

### Purification 3_Ion exchange chromatography

### <8-1> Ion exchange chromatography

Buffer I (4 M Urea, 20 mM Na-AcOH, pH 4.0±0.2) was injected into the column in an amount of 2 CV or more to perform equilibration. The entire amount of the Purification 2 eluate was injected into the column. An unbound wash was performed by injecting 5 CV of Buffer I into the column.

Washing was performed by injecting 7 CV of Buffer J (4 M Urea, 20 mM Na-AcOH, 0.2 M NaCl pH 4.0±0.2) into the column.

Elution was performed by injecting 5 CV of Buffer K (4 M Urea, 20 mM Na-AcOH, 0.5 M NaCl pH 4.0±0.2) into the column. The entire amount of 5 CV was recovered as the eluate from the time of injecting Buffer K. When the collection was completed during elution, it was possible to proceed directly to the column regeneration and storage steps. At the end of elution, 5 mL of the elution fraction was divided into 15 mL conical tubes and used for the following process solution analyses: purity (SDS-PAGE), purity (RP-HPLC) (IPC), and concentration (660 nm protein assay).

### <8-2> Final yield after final purification

The final yield of TGF-β3 obtained after the final third purification process was a TGF-β3 protein concentration of 0.977 g/L and a TGF-β3 protein amount of 715.2 mg when the process solution volume was 0.732 L.

### [Example 9]

### Filtration

A membrane (10 kDa, 0.1 m²*1 sheet) was mounted in an ultrafiltration/diafiltration system (UF/DF system).

Equilibration was performed by injecting Buffer L (20 mM Na-OAc, pH 3.8±0.2) into the membrane. Equilibration was completed when the pH of the permeate/filtrate was 3.8±0.3 and the electrical conductivity was 2 bar or less.

Filtration for concentration (Ultrafiltration) was performed under the condition of a transmembrane pressure (TMP) ([P_{Feed}+P_{Ret}]/2) of 2 bar or less.

The concentration volume was calculated by [Mathematical Formula 3] below. The set diafiltration substance (DS) concentration was 1.0 g/L, but considering the difference between the Purification 3 eluate and the DS concentration analysis method, concentration was performed up to a concentration twice the set concentration and then diluted. Concentration volume = rhTGF-β3 protein content (g) in Purification 3 eluate/2.0 g/L

Buffer L corresponding to the volume after concentration was injected to perform buffer exchange (diafiltration). When the pH of the permeate/filtrate was 3.8±0.2 at the end of six cycles of diafiltration, the filtration process was terminated. When it was not suitable, diafiltration was performed for one further cycle, and it was confirmed whether the permeate/filtrate at the end of each cycle met the pH standard.

Additional concentration was performed for line recovery. After closing the permeate valve, Buffer L was used to perform line recovery.

The concentration of the recovery solution was measured using the A280 method, and the volume of Buffer L to be added so that the final concentration of the stock solution was 1.0 g/L was calculated and added. For the measurement of the A280 protein concentration, 500 µL of DS was added to a cuvette, and the absorbance was measured at 280 nm using a multi-spectrophotometer (ELISA reader). When the measured absorbance was outside the range of 0.3 to 0.7, the dilution factor was adjusted so that the absorbance fell within the range. The measured absorbance was multiplied by the dilution factor and divided by the extinction coefficient of 1.841 to calculate the protein concentration. Dilution was performed with the calculated volume of Buffer L, and the recovered solution whose final concentration was confirmed using the A280 method was filtered using a 0.2 µm bottle top filter.

The filtrate whose concentration was confirmed was filtered into a sterilized glass bottle using a 0.2 µm bottle-top filter (polyethersulfone (PES)), and the final solution was divided into 50 1 mL aliquots in 1.5 mL EP tubes and used for the lot release test.

### [Example 10]

### Specification Evaluation

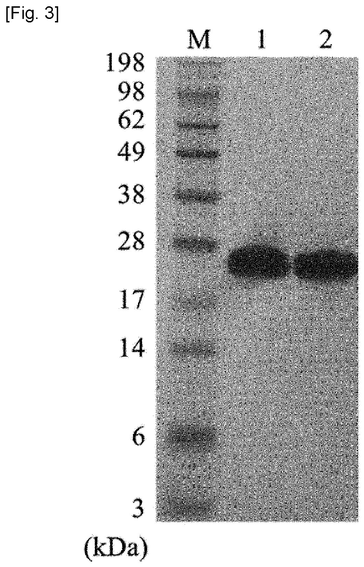

Specification evaluation was conducted on the TGF-β3 finally isolated and purified through the processes of [Example 1] to [Example 9], and the results are shown in [Table 5] below. In particular, the electrophoresis results were consistent with the TGF-β3 standard solution (lane 1), and no additional impurity proteins were identified, confirming that the target protein, TGF-β3 (lane 2), was purified with high purity (FIG. 3).

**[Table 5]**

| Test item | Criteria | Test result | Judgment |
|---|---|---|---|
| Characteristics | Colorless and clear to slightly opaque solution | A colorless transparent solution | Suitable |
| pH | 3.8 ± 0.5 | 3.9 | Suitable |
| Endotoxin | 125.0 EU/mg or less | 0.1 EU/mg | Suitable |
| Electrophoresis | When compared to the main band of the standard solution, the main band of the test solution should be identified at the same position. | When compared to the main band of the standard solution, the main band of the test solution was identified at the same position. | Suitable |
| Western blotting | When compared to the main band of the standard solution, the main band of the test solution should be identified at the same position. | When compared to the main band of the standard solution, the main band of the test solution was identified at the same position. | Suitable |
| Protein quantification (A280) | Measured value (mg/mL) | 0.977 | Suitable |
| Activity (Cell assay) | 1.9 x 10⁷ IU/mg or more | 2.394 x 10⁷ IU/mg | Suitable |
| Purity (RP-HPLC) | The %Area of the main peak should be 95.0% or more. | 99.9% | Suitable |
| Purity (SEC-HPLC) | The %Area of the main peak should be 95.0% or more. | 100.0% | Suitable |
| Host Cell Protein (HCP) | 100 ppm or less | 4 | Suitable |

### [Industrial Applicability]

Since the pretreatment method of the present invention and the purification method including the same utilize *E. coli,* the process is simple and high productivity can be expected compared to an animal cell culture method, so that high-purity TGF-β3 protein can be isolated and purified with high efficiency from *E. coli,* and thus the pretreatment method and the purification method have industrial applicability.

## Claims

1. A pretreatment method for purifying TGF-β3 protein, comprising:
i) a step of recovering *E. coli* from an *E. coli* culture medium;
ii) a step of disrupting the recovered *E. coli* to obtain a TGF-β3 inclusion body;
iii) a step of washing the inclusion body;
iv) a step of solubilizing the washed inclusion body; and
v) a step of refolding the solubilized inclusion body.

2. The method of claim 1, wherein the disrupting in Step ii) is performed one to five times at a pressure of 800 to 1200 bar.

3. The method of claim 1, wherein the washing in Step iii) is performed one to five times.

4. The method of claim 1, wherein the washing in Step iii) includes a step of washing with a surfactant and then washing with distilled water.

5. The method of claim 1, wherein the solubilizing in Step iv) is performed with a buffer containing one or more selected from the group consisting of urea, 1,4-dithiothreitol (DTT), and tris(hydroxymethyl)aminomethane.

6. The method of claim 1, wherein the solubilizing in Step iv) is performed with a buffer having a pH of 7 to 9.

7. The method of claim 1, wherein the solubilizing in Step iv) is performed with a buffer for 1 to 24 hours.

8. The method of claim 1, wherein the refolding in Step v) is performed with a buffer containing one or more selected from the group consisting of (N-cyclohexyl-2-aminoethanesulfonic acid (CHES), NaCl, reduced glutathione, oxidized glutathione, 3-[(3-cholamidopropyl)dimethylammonio]propane-1-sulfonate (CHAPS), L-arginine, and D-sorbitol.

9. The method of claim 1, wherein the refolding in Step v) is performed with a buffer having a pH of 8.5 to 10.5.

10. The method of claim 1, wherein the refolding in Step v) is performed with a buffer for 1 to 12 days.

11. The method of claim 1, wherein the refolding in Step v) is performed so that the final concentration becomes 0.1 to 2.0 g/L.

12. A TGF-β3 protein purification method comprising:
i) a step of recovering *E. coli* from an *E. coli* culture medium;
ii) a step of disrupting the recovered *E. coli* to obtain a TGF-β3 inclusion body;
iii) a step of washing the inclusion body;
iv) a step of solubilizing the washed inclusion body;
v) a step of refolding the solubilized inclusion body;
vi) a step of purifying a solution containing the refolded TGF-β3; and
vii) a step of filtering the purified solution.

13. The method of claim 12, wherein the purifying in Step vi) includes:
a) a step of performing hydrophobic interaction chromatography as primary purification;
b) a step of performing multimodal chromatography as secondary purification; and
c) a step of performing cation exchange chromatography as a tertiary purification.

14. The method of claim 12, wherein the filtering in Step vii) is ultrafiltration/diafiltration.
